# EUROPEAN PATENT APPLICATION

(11) **EP 3 680 345 A1**
(43) Date of publication of application: **15.07.2020**
(21) Application number: 18853141.2
(22) Date of filing: 23.08.2018
(51) Int. Cl.: C12Q 1/68

(54) **POROUS MATERIAL FOR THE DETECTION OF CANDIDA ALBICANS, DIAGNOSTIC METHOD USING SAME AND PREPARATION METHOD THEREOF**

(30) Priority: 05.09.2017 ES 201731069
(71) Applicant: Universitat Politècnica de València, 46022 Valencia (ES); Consorcio Centro de Investigación Biomédica en Red, M.P., 28029 Madrid (ES); Instituto de Investigacion Sanitaria la Fe-Fundacion Para la Investigacion Del Hospital Universitario Y Politecnico la fe de la, 46026 Valencia (ES); Universitat Rovira I Virgili, 43003 Tarragona (ES)
(72) Inventor: RIBES MONPARLER, Àngela, 46002 Valencia (ES); AZNAR GIMENO, Elena, 46022 Valencia (ES); MARTÍNEZ MÁÑEZ, Ramón, 46002 Valencia (ES); SANCENÓN GALARZA, Félix, 46002 Valencia (ES); MARCOS MARTÍNEZ, María Dolores, 46002 Valencia (ES); TORMO MAS, María Ángeles, 46026 Valencia (ES); PEMÁN GARCÍA, Javier, 46026 Valencia (ES); MARSAL GARVI, Lluis Francisco, 43003 Tarragona (ES); XIFRÉ PÉREZ, Elisabet, 43003 Tarragona (ES)
(74) Representative: Isern Patentes y Marcas S.L.
(86) International application number: PCT/ES2018/070571
(87) International publication number: WO 2019/048722

(57) **Abstract**

Specifically, the invention describes obtaining a new porous material prepared to recognise DNA of the pathogenic microorganism *Candida albicans,* as well as the use thereof in a quick and highly sensitive in vitro diagnostic method.

## Description

### Field of the invention

The present invention belongs to the field of new materials with applications in the detection of species of biological, pharmacological and medical interest, which can be applied to in situ diagnostic methods. Specifically, the invention discloses obtaining a new porous material prepared to recognise DNA of the pathogenic microorganism *Candida albicans,* as well as the use thereof in a quick and highly sensitive in vitro diagnostic method.

### Background of the invention

In recent years there has been significant growth in the development of organic-inorganic hybrid materials with multiple applications in different scientific and technological fields. Among these hybrid materials, those which have been developed by taking into account the "molecular gate" concept have stood out due to the potential applications thereof in processes for controlled release and molecular identification. A functionalised hybrid material with "molecular gates" is composed of a porous support which is capable of storing certain types of molecules/species in the inner pores thereof and also has an organic or inorganic group, which may be molecular or supramolecular, anchored on the outer surface thereof, wherein said group is capable of modulating the release of the molecules stored in the inner pores thereof through the application of an external stimulus, known as loading the support. These materials represent a very promising technology and have been intensively researched in recent years in fields such as bioengineering, biodetection and bionanotechnology, thereby opening new horizons.^{1,2} The external stimuli most commonly used to control the opening/closing of the "molecular gates" can be classified as: photochemical, electrochemical, ionic (changes in pH, presence of certain cations and anions), changes in temperature, changes in polarity, or the presence of certain molecules or biomolecules (enzymes, antigen/antibody systems, single DNA strands, etc.).³⁻⁵ The application of these external stimuli can induce the release of an indicator species (dyes, fluorophores, substances with redox activity, plasmon resonance or that are biologically active such as cytotoxic agents, proteins, small biomolecules, enzymes or nucleic acid fragments), in the right place and time and in a controlled manner.⁶⁻¹² While this application is very important, few examples of the application organic-inorganic hybrid materials functionalised with "molecular gates" have been described in recognition and detection protocols.^{13-15a and 15b}

In order to apply hybrid materials with "molecular gates" to detection methods, the pores of the inorganic support are usually loaded with an indicator species and the outer surface of the solid is functionalised with (bio)molecules, supramolecules or inorganic particles. The functionalisation of the support refers to the bonding or anchoring on the outer surface of the support of certain (bio)molecules, supramolecules or inorganic particles that interact with a certain target analyte, inducing a spatial change at the inlet of the pores of the support; therefore, in the absence of the target analyte, the "molecular gate" will be closed, since the biomolecules, supramolecules or inorganic particles anchored on the outer surface of the support impede or inhibit the release of the molecules contained in the inner pores. However, in the presence of the target analyte, it will induce changes in the structures/conformations of the (bio)molecules, supramolecules or inorganic particles which act as a "molecular gate", and the load of the inner pores will be released. This release into the solution will be reflected in a physical, chemical or biological change (for example, in color or fluorescence) which can be easily quantified.^{16,17} This new concept of detection differs from the classical "coordinating subunit-indicator subunit" supramolecular system, since the recognition process is separated from the signalling process.¹⁸⁻²⁴ Furthermore, another advantage of this new protocol is the amplification of the signal. For example, in these materials it has been described that the presence of few analyte molecules can induce the release of large amounts of indicator species from the inside of the pores of the support.²⁵

Moreover, in last few decades, cases of nosocomial infections caused by yeasts, such as candidiasis, have increased in hospitalised patients.²⁶ There are various potentially dangerous strains of *Candida,* although the most invasive infections are those caused by *Candida albicans.* Therefore, it is very important and of great interest to quickly and unequivocally diagnose cases of infection caused by this species. Furthermore, it is also important to be able to differentiate between *Candida albicans* and other species due to the different treatments that must be followed in each case. Indeed, *Candida albicans* has an intrinsic resistance to the most commonly used antimicrobial agent, Fluconazole.²⁷ Currently, there are reliable methods for detecting *Candida albicans,* however, they are slow, expensive and dependent on specialised laboratories with specifically trained staff.²⁸⁻³⁰

### References

1. E. Katz et al, Integrated nanoparticle-biomolecule hybrid systems: synthesis, properties, and applications, Angew. Chem. Int. Ed. 2004, 43, 6042-6108.
2. F. Wang, et al, DNA switches: from principles to applications, Angew. Chem. Int. Ed. 2015, 54, 1098-1129.
3. A. Bernardos, et al, Enzyme-responsive intracellular controlled release using nanometric silica mesoporous supports capped with "saccharides", ACS Nano, 2010, 4(11), 6353-6368.
4 A. Bernardos, et al, Enzyme-responsive controlled release using mesoporous silica supports capped with lactose, Angew Chem Int Ed Engl. 2009; 48(32): 5884-5887.
5. N. Mas, et al, Enhanced antifungal efficacy of tebuconazole using gated pH-driven mesoporous nanoparticles, Int. J. Nanomedicine. 2014, 9, 2597-2606.
6. E. Aznar, Gated Materials for On-Command Release of Guest Molecules, Chem. Rev., 2016, 116, 561-718.
7. A. B. Descalzo, et al, The supramolecular chemistry of organic-inorganic hybrid materials, Angew. Chem. Int. Ed., 2006, 45, 5924.
8. R. Casasús, et al, Dual Aperture Control on pH- and Anion-Driven Supramolecular Nanoscopic Hybrid Gate-like Ensembles, J. Am. Chem. Soc., 2008, 140, 1903-1917.
9. S. Angelos, et al, pH-responsive supramolecular nanovalves based on cucurbit [6]uril pseudorotaxanes, Angew. Chem. Int. Ed., 2008, 47, 2222-2226.
10. C. Park, et al, Controlled Release of Guest Molecules from Mesoporous Silica Particles Based on a pH-Responsive Polypseudorotaxane Motif, Angew. Chem. Int. Ed., 2007, 46, 1455-1457.
11. M. Vallet-Regi, et al, A New Property of MCM-41: Drug Delivery System, J. Chem. Mater., 2001, 13, 308-311.
12 M. Vallet-Regi, et al, Arcos, Mesoporous materials for drug delivery, Angew. Chem. Int. Ed. 2007, 46, 7548-7558.
13. F. Sancenón, et al, Gated Silica Mesoporous Materials in Sensing Applications, ChemistryOpen, 2015, 4, 418-437.
14. Y. Salinas, et al, Chromo-Fluorogenic Detection of Nitroaromatic Explosives by Using Silica Mesoporous Supports Gated with Tetrathiafulvalene Derivatives, Chem. Eur., J. 2014, 20, 855-866.
15a E. Climent, et al, Controlled Delivery Using Oligonucleotide-Capped Mesoporous Silica Nanoparticles, Angew. Chem. 2010, 122, 7439 -7441.
15b LI. Pascual, et al, Oligonucleotide-capped mesoporous silica nanoparticles as DNA-responsive dye delivery systems for genomic DNA detection, Chem. Commun., 2015, 51, 1414-1416.
16. C. Coll, et al, Gated Silica Mesoporous Supports for Controlled Release and Signaling Applications, Acc. Chem. Res., 2013, 46, 339-349.
17. E. Climent, et al, Selective, Highly Sensitive, and Rapid Detection of Genomic DNA by Using Gated Materials: Mycoplasma Detection, Angew. Chem. Int. Ed. 2013, 52, 8938-8942.
18. L. E. Santos-Figueroa, et al, Chromogenic and fluorogenic chemosensors and reagents for anions. A comprehensive review of the years 2010-2011, Chem. Soc. Rev., 2013, 42, 3489-3613.
19. M. E. Moragues, et al, Chromogenic and fluorogenic chemosensors and reagents for anions. A comprehensive review of the year 2009, Chem. Soc. Rev., 2011, 40, 2593-2643.
20. Y. Salinas, et al, Optical chemosensors and reagents to detect explosives, Chem. Soc. Rev. 2012, 41, 1261-1296.
21. S. K. Kim, et al, Chemosensors for pyrophosphate, Acc. Chem. Res., 2009, 42, 23 - 31.
22. X. Chen, et al, Fluorescent and colorimetric probes for detection of thiols, Chem. Soc. Rev., 2010, 39, 2120 - 2135.
23. A. P. de Silva, et al, Signaling Recognition Events with Fluorescent Sensors and Switches, Chem. Rev., 1997, 97, 1515 - 1566.
24. R. Martínez-Máñez et al, Fluorogenic and Chromogenic Chemosensors and Reagents for Anions, Chem. Rev. 2003, 103, 4419 - 4476.
25. M. Hecht, et al, Gated hybrid delivery systems: En route to sensory materials with inherent signal amplification, Coord. Chem. Rev., 2013, 257, 2589 -2606.
26. S.K. Fridkin, The changing face of fungal infections in health care settings, Healthcare Epidemiology, 2005, 41, 1455-1460.
27. A. Vahidnia, et al, High throughput multiplex-PCR for direct detection and diagnosis of dermatophyte species, Candida albicans and Candida parapsilosis in clinical specimen, J. Microbiol. Methods, 2015, 113, 38 - 40.
28. Y. Guo, et al, A Real-Time PCR Assay Based on 5.8S rRNA Gene (5.8S rDNA) for Rapid Detection of Candida from Whole Blood Samples, Mycopathologia, 2016, 181, 405-413.
29. J. Kim, et al, Evaluation of the Punch-it™ NA-Sample kit for detecting microbial DNA in blood culture bottles using PCR-reverse blot hybridization assay, J. Microbiol. meth, 2016, 128, 24-30.
30. X. Jiang, et al, Rapid Detection of Candida albicans by Polymerase Spiral Reaction Assay in Clinical Blood Samples, Front. Microbiol., 2016, 7, 916.
31. Y. H. Lim and D. H. Lee, Rapid PCR Method for Detecting Candida albicans Using Primers Derived from the Integrin-like Protein Gene αINT1 of Candida albicans, J. Microbiol, 2000, 38, 105-108.
32. E. P. Barrett, et al, The Determination of Pore Volume and Area Distributions in Porous Substances. I. Computations from Nitrogen Isotherms, J. Am. Chem. Soc. 1951, 73, 373-380.
33. S. Brunauer, et al, Adsorption of Gases in Multimolecular Layers, J. Am. Chem. Soc. 1938, 60, 309-319.

### General description of the invention

The authors of the present invention have obtained a new porous material for detecting *Candida albicans,* and they have developed a preparation method thereof and an in vitro diagnostic method that uses it.

A first aspect of the present invention relates to a support made of porous material for detecting *Candida albicans* which comprises an indicator species in the inner pores and at least one DNA sequence which is complementary to a fragment of the *Candida albicans* genome, wherein the complementary DNA sequence blocks the release of the indicator species from the inner pores of the support.

In the present invention, the DNA sequence complementary to a fragment of the *Candida albicans* genome which is anchored on the outer surface of the porous material and blocks the release of the indicator species from the inner pores of said material, together with the organic structures (neutral or cationic organic groups) that bond it to the porous material, what is currently known as "molecular gates". Given the three-dimensional structure and conformation of the complementary DNA sequence, this sequence blocks the pores of the porous material, thereby impeding the release of the indicator species.

Said DNA sequences are complementary to a sequence selected from the genome of *Candida albicans.* In a particular embodiment of the present invention, the complementary DNA sequence blocking the pores is complementary to a sequence of the gene *αINT1* of *Candida albicans.*³¹

In a particular embodiment of the present invention, the complementary DNA sequence is bonded to the outer surface of the support by means of a neutral organic group and a bonding oligonucleotide that hybridises with the complementary DNA of *Candida albicans.* In a more particular embodiment, the complementary DNA sequence that hybridises with the bonding oligonucleotide is oligonucleotide O2 (SEQ No. 2), which is complementary to a sequence of the gene *αINT1* of *Candida albicans.* In a more particular embodiment, the bonding oligonucleotide is oligonucleotide O1 (SEQ No. 1). In a more particular embodiment, oligonucleotide O1 (SEQ No. 1) is bonded to an outer surface of the support by means of a neutral organic group. In an even more particular embodiment, the neutral organic group is selected from carboxylic acid (-COOH), alcohol (-OH), aldehyde (-CHO), alkene, alkyne, amine (-NH2 or - NR'R"), amide (-C(O)NR'R"), azide (-N3), ketone (-C=O), ester (-COOR'), ether (R'-O-R"), halogen, imine (RR'C=NR"), isocyanate (-NCO), isothiocyanate (-N=C=S), nitrile (-C=N), nitro (-NO2) or thiol (-SH). In a preferred embodiment, the neutral organic group is an isocyanate group (-NCO).

In an another particular embodiment of the present invention, the complementary DNA sequence is bonded to an outer surface of the support by means of a cationic organic group. In a more particular embodiment, the complementary DNA sequence that is bonded to the cationic organic group is oligonucleotide O3 (SEQ No. 3), which is complementary to a sequence of the gene *αINT1* of *Candida albicans.* In a more particular embodiment, the cationic inorganic group is selected from amines, guanidine groups (H-N=(NHR)NH2), phosphonium (PH₄⁺) or quaternary ammonium (NR₄⁺). R is selected from linear or branched C₁-C₆ alkyl and C₃-C₆ cycloalkyl.

In one embodiment of the present invention, the DNA sequence is selected from:
Oligonucleotide O1-5'-AAA AAA CCC CCC-3' (SEQ No. 1)
Oligonucleotide O2-5'-TTT TGG GGG GTT GAG AAG GAT CTT TCC ATT GAT GGG GTT TT-3' (SEQ No. 2)
Oligonucleotide O3-5'-TTG AGA AGG ATC TTT CCA TTG ATG-3' (SEQ No. 3).

In a more particular embodiment of the present invention, the complementary DNA sequence is selected from:
Oligonucleotide 2-5'-TTT TGG GGG GTT GAG AAG GAT CTT TCC ATT GAT GGG GTT TT-3' (SEQ No. 2)
Oligonucleotide 3-5'-TTG AGA AGG ATC TTT CCA TTG ATG-3' (SEQ No. 3).

In the present invention, the porous material is selected from mesoporous silicon dioxide in the form of nanoparticles, also known as mesoporous silicon nanoparticles (MSNs) or nanoporous anodised alumina (AAN) in the form of a film or plate. More particularly, the porous material of the present invention has a specific surface between 200-1,400 m²g⁻¹.

In a particular embodiment of the present invention, the method for preparing the porous material comprises the following steps:
a) Preparing a support made of porous silicon dioxide or nanoporous anodised alumina,
b) Introducing the indicator species into the inner pores of the substrate prepared in step a),
c) Functionalising the loaded substrate obtained in step b) by bonding a neutral or cationic organic group to the surface of said substrate,
d) If necessary, derivatising the substrate with a bonding oligonucleotide bonded to the neutral organic group,
e) Adding a DNA sequence which is complementary to a fragment of the *Candida albicans genome* to the porous inorganic support obtained in step c) or d).

In an another particular embodiment of the present invention, the in vitro diagnostic method for detecting *Candida albicans* comprises:
a) putting the support made of porous material in contact with the DNA sample to be analysed in an aqueous solution,
b) incubating the mixture of step a) so as to produce the hybridisation reaction,
c) measuring the amount of indicator species released into the aqueous phase of the mixture.

When the porous material is put in contact with a sample containing the genomic DNA of *Candida albicans,* in an aqueous solution, it will become hybridised with the DNA sequence which is used as a "molecular gate", with the ensuing opening or unblocking of the pores and release of the indicator species housed in the interior of said porous material. These materials will be capable of selectively detecting the presence of *Candida albicans,* thereby constituting a quick, simple and highly sensitive sensing system.

This new porous material for in vitro diagnosis of the infection caused by *Candida albicans* has high sensitivity and specificity, a low manufacturing cost and enables the quick detection of the infection at the very doctor's office where the sample is taken from the patient, thereby minimising dependence on specialised laboratories and specifically trained laboratory staff.

Additionally, the following advantages can be proposed:
- Facilitating medical diagnosis and decision-making, through the use of a fast, powerful tool for detecting (in vitro diagnostic method) a *Candida albicans* infection.
- Reducing diagnosis time from 3-4 days to 10-15 minutes.
- Lowering the cost of the diagnostic test up to 5 times its current value.
- Limiting dependence on specialised laboratories to detect the presence of *Candida albicans* in a sample from a patient.
- Diagnosing *Candida albicans* at the doctor's office where the sample is taken from the patient.
- Applying supramolecular chemistry concepts and knowledge to the design of nanoscopic devices aimed at meeting the clinical needs of patients.
- Providing the domestic and international market with an innovative technology capable of revolutionising the healthcare industry with a probe material for detecting *Candida albicans,* which is responsible for a high percentage of infections in the world.

In the present invention, the term "functionalised", as used in the present specification, refers to the chemical modification of the porous support with neutral or organic functional groups, as mentioned earlier.

In the present invention, the term "in vitro diagnostic method", as used in the present specification, refers to a protocol for the sensitive and selective detection of *Candida albicans.*

In the present invention, the term "loaded", as used in the description, refers to the incorporation of the indicator species inside the pores of the porous support by means of a diffusion or impregnation process.

In the present invention, the term "indicator species" refers to any species susceptible to being quantified, such as dyes, fluorophores, substances with redox activity, plasmon resonance or that are biologically active such as cytotoxic agents, proteins, small biomolecules, enzymes or nucleic acid fragments.

In the present invention, the term "derivatisation" refers to the modification of the organic group anchored on the porous surface with the bonding oligonucleotide through the formation of a chemical bond.

### Industrial applicability

The new method for in vitro diagnosis of the infection caused by *Candida albicans* constitutes a solid proposal that potentially improves the positioning of the Health Technology sector and the companies in it, creating new opportunities in the sector. Thanks to the use of this new detection tool, the costs and resources currently dedicated to the detection of this infection can be considerably reduced.

### Brief description of the figures

Figure 1 shows the diagram of material S1. In this case, the porous material is loaded with the indicator species (dye/fluorophore) and functionalised with a neutral organic group.
Figure 2 shows the diagram of material S2. In this case, the porous material is loaded with the indicator species (dye/fluorophore), functionalised with a neutral organic group, and wherein oligonucleotide O1 (SEQ No. 1) is covalently anchored by means of a urea functional group.
Figure 3 shows the diagram of material S3. In this case, the porous material is loaded with a dye/fluorophore and functionalised with oligonucleotide O1 (SEQ No. 1) whereon oligonucleotide O2 (SEQ No. 2) is hybridised.
Figure 4 shows the diagram of material S4. In this case, the porous material is loaded with a dye/fluorophore and the outer surface is functionalised with a cationic organic group.
Figure 5 shows the diagram of material S5. In this case, the porous material is loaded with a dye/fluorophore and functionalised with oligonucleotide O3 (SEQ No. 3).
Figure 6 shows:
   - I) X-ray powder diffractogram for: a) non-calcined MSNs, b) calcined MSNs, c) S1 (which is equal to S4), d) material S2, and e) material S3 (equal to S5).
   - II) Transmission Electron Microscopy images for: A) calcined MSNs and B) material S3, S5 showing the spherical morphology typical of nanoparticles.

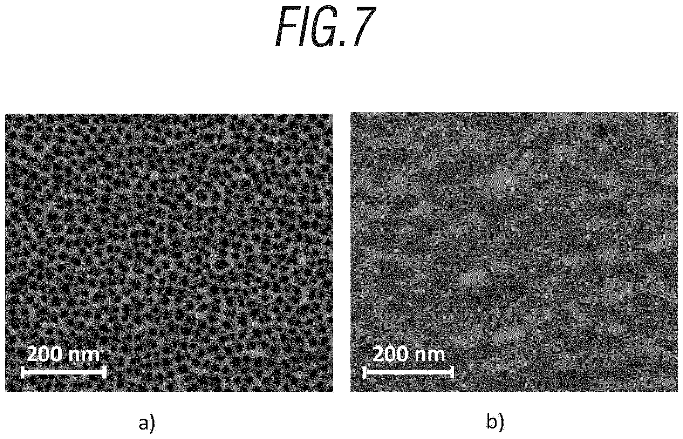

Figure 7 shows Field Emission Scanning Electron Microscope (FESEM) images of:
   a) non-functionalised porous alumina, and
   B) oligonucleotide-functionalised porous alumina (which can be anchored on the support by means of covalent bonds (S8) or electrostatic interactions (S10). In both images, the disordered pore system of the inorganic matrix can be observed.

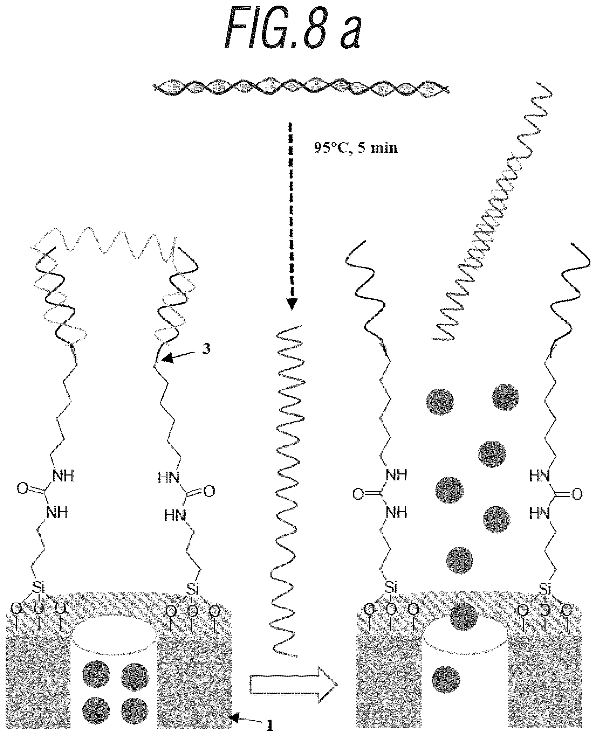

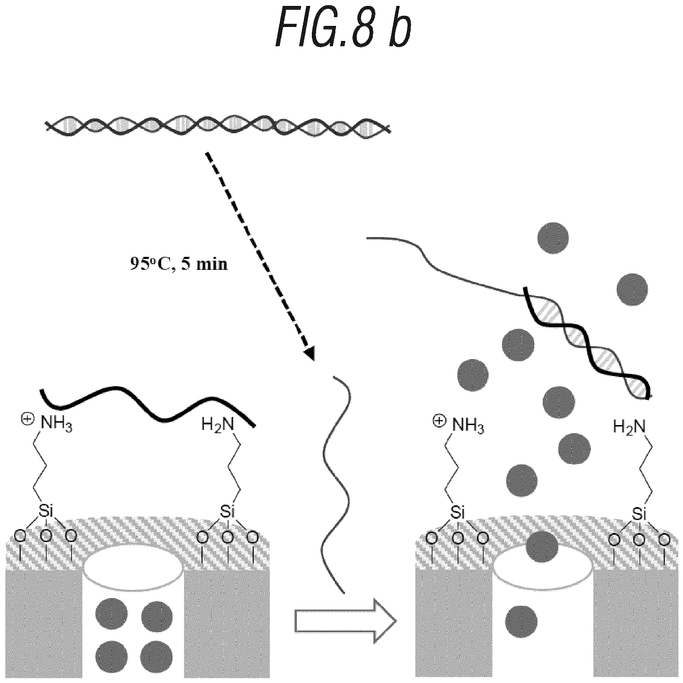

Figure 8 (A and B) shows the functional diagram of materials S3 and S5 in the presence of genomic DNA of *Candida albicans.*

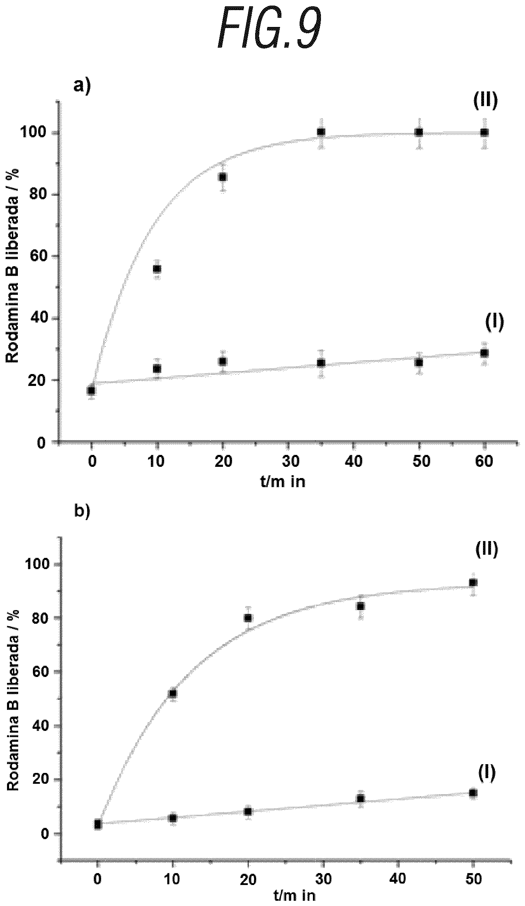

Figure 9 shows the dye/fluorophore release curves (measured by means of typical fluorescence emission of Rhodamine B at 575 nm exciting at 555 nm) from inside the pores of materials S3 (a) and S5 (b) in the absence (I) and presence (II) of genomic DNA of *Candida albicans.*

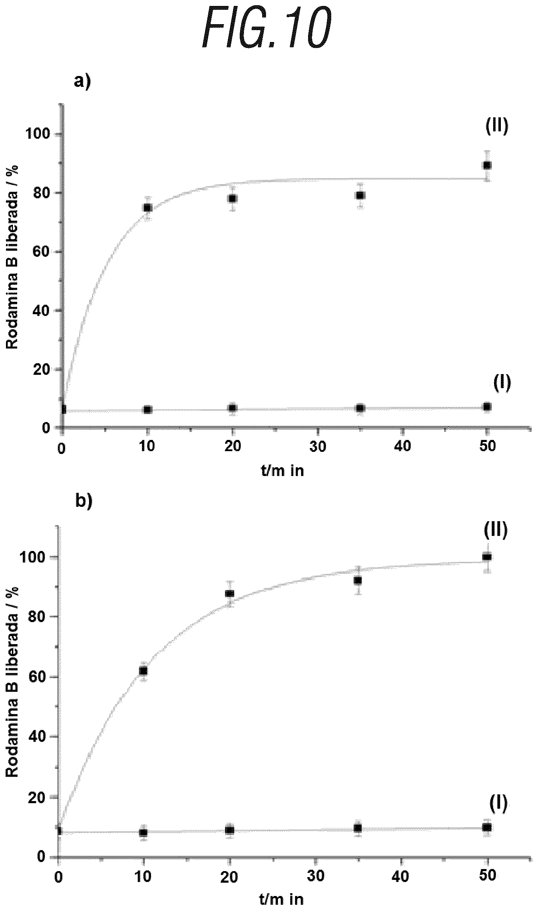

Figure 10 shows the dye/fluorophore release curves (measured by means of typical fluorescence emission of Rhodamine B at 575 nm exciting at 555 nm) from inside the pores of materials S8 (a) and S10 (b) in the absence (I) and presence (II) of genomic DNA of *Candida albicans.*

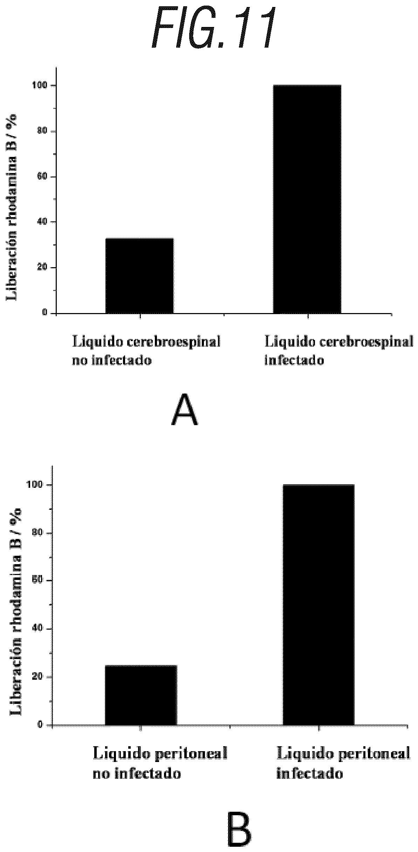

Figure 11 shows the fluorescence (measured at 575 nm) of Rhodamine B released from material S8 in the presence of genomic DNA of *Candida albicans* in real samples from infected patients (A Cerebrospinal fluid, B Peritoneal fluid).

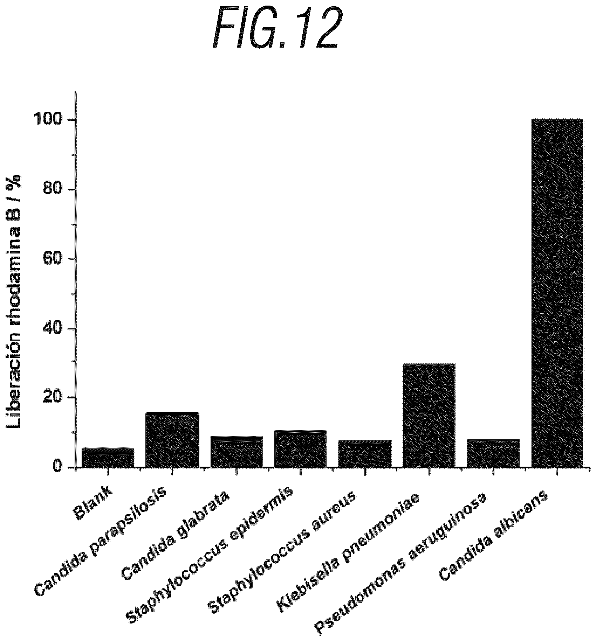

Figure 12 shows the selectivity of material S8. The fluorescence intensity of Rhodamine B released from S8 in the presence of different pathogens and other strains of *Candida* is shown.
The present invention will be additionally illustrated by the following examples. The examples are provided for illustrative purposes and do not limit the scope of the invention in any way.

### Materials and Instrumentation

A magnetic stirrer and normal glass and plastic material were used to prepare the porous materials. A Hettich Rotina 35 centrifuge was used to separate the materials when these were in suspension. All the reagents used to prepare the materials were used as received from the commercial firms without any additional purification.

The materials obtained were characterised by means of X-ray Powder Diffraction, Transmission Electron Microscopy (TEM), Field Emission Scanning Electron Microscopy (FESEM), N₂ adsorption-desorption isotherms, particle size by means of Dynamic Light Scattering (DLS), Fourier-Transform Infrared Spectroscopy (FTIR), thermogravimetric analysis and fluorescence spectroscopy. An Advance D8 diffractometer was used in the measurement of X-ray Powder Diffraction, using CuKα radiation (Philips, Amsterdam, the Netherlands). The TEM images were obtained using a CM10 100kV microscope (Philips). The FESEM images were obtained using an ULTRA 55 microscope (Zeiss). An automatic ASAP 2010 adsorption analyser (Micrometrics, Norcross, GA, USA) was used in the measurement of nitrogen adsorption-desorption isotherms.

The samples were degasified at 120°C in a vacuum overnight. The specific surface was calculated based on the adsorption data obtained in the low-pressure range using the Brunauer-Emmett-Teller (BET) model.³² Pore size was determined following the Barret-Joyner-Halenda (BJH) method.³³ The distribution of the particle size of the different solids was obtained using a Zetasizer Nano unit (Malvern Instruments, Malvern, UK). For these measurements, the samples were dispersed in deionised water. Data analysis was based on the Mie Theory, using refractive indices of 1.33 and 1.45 for the dispersant and for the nanoparticles, respectively. An adsorption value of 0.001 was used for all the samples. All the measurements were made in triplicate.

The infrared spectra were obtained using a Bruker TENSOR II spectrophotometer with Platinum ATR. Thermogravimetric analysis was carried out using a TGA/SDTA 851e balance (Mettler Toledo, Columbus, OH, USA) using an oxidising atmosphere (air, 80 ml/min) with a temperature program which consisted of a gradient of 393-1273K at 10°C/min, followed by an isotherm at 1273K for 30 min. The fluorescence measurements were made in a Felix 32 Analysis version 1.2 fluorimeter (Build 56, Photon Technology International, Birmingham, NJ, USA).

### Examples of embodiment

### Example 1: Synthesis of mesoporous silicon nanoparticles (MSNs)

Mesoporous silicon nanoparticles (MSNs) were synthesised using a surfactant (hexadecyltrimethylammonium bromide, CTABr) and a source of silicon (tetraethoxysilane, TEOS). To this end, the CTABr (1.00 g, 2.74 mmol) was first dissolved in 480 ml of deionised water. Next, NaOH (2.00 M, 3.50 ml) was added to the previous solution and the temperature was adjusted to 80°C. Next, TEOS was added (5.00 ml, 25.7 mmol) drop by drop to the previous solution. The mixture was kept under stirring for 2 hours, obtaining a white precipitate. The resulting powder was centrifuged and washed with deionised water. Finally, the solid was dried at 60°C. To prepare the final porous support, the material was calcined at 550°C using an oxidising atmosphere for 5 hours in order to remove the surfactant.

### Example 2: Synthesis of material S1

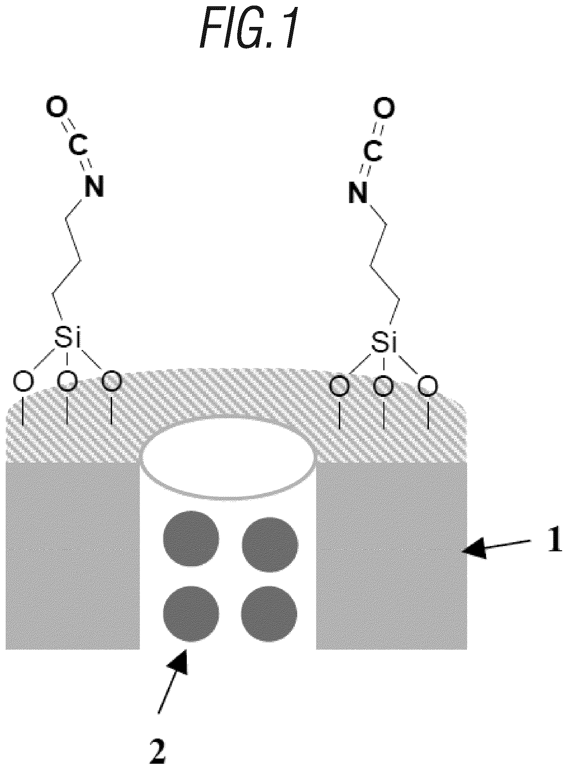

200 µg of calcined MSNs (50-150 nm in diameter, 1,200 m²/g of total surface) were suspended in a solution containing 766.4 mg (0.16 mmol) of Rhodamine B in 10 ml of acetonitrile, leaving the mixture under stirring at room temperature for 24 hours. Next, 247 µl (1 mmol) of (3-propyl isocyanate) triethoxysilane were added and left to react at room temperature for 5.5 hours. Next, the mixture was vacuum filtered, washed with acetonitrile and dried in a heater at 65°C. The solid obtained was characterised using thermogravimetry, TEM and X-ray Powder Diffraction techniques and identified as material S1. Figure 1 shows the mesoporous inorganic matrix (1) obtained, which is loaded with a dye/fluorophore (Rhodamine B) (2) and functionalised with a neutral organic group.

### Example 3: Synthesis of material S2

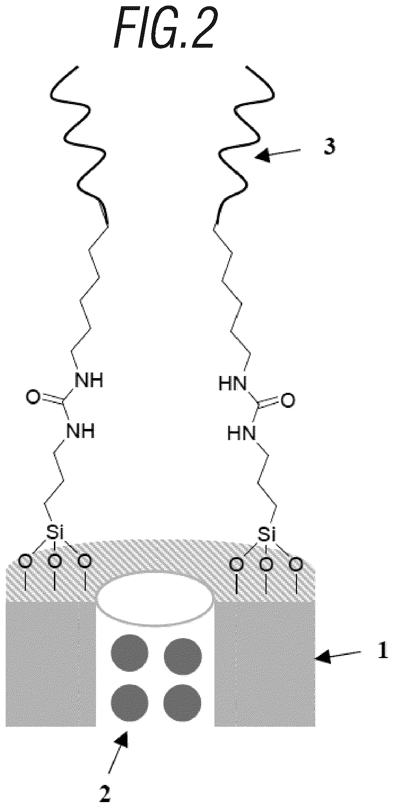

1 mg of material S1 was suspended in 700 µl of an acetonitrile solution with Rhodamine B (1 mM), adding oligonucleotide O1 (SEQ No. 1) (NH₂-(CH₂)₆-5'-AAA AAA CCC-3') in a concentration of 1 nmol/mg of solid. 2 µl of triethylamine were added to this mixture and left under stirring at room temperature for 3 hours. Next, the suspension was centrifuged for 2 min at 12,000 rpm and the liquid was separated, washing the solid with 1 ml of an aqueous buffer solution at pH 7.5 (MgCl₂ 37.5 mM, Tris-HCI 20 mM). This suspension was separated again by centrifugation, thus obtaining material S2. Figure 2 shows the solid obtained, which was characterised by using thermogravimetry, TEM and X-ray Powder Diffraction techniques. As shown in said Figure 2, the porous material (1) is loaded with a dye/fluorophore (Rhodamine B) (2) and functionalised with oligonucleotide O1 (SEQ No. 1) (3), which is covalently anchored by means of a urea functional group. The concentration of oligonucleotide O1 in material S2 is 1 nmoles/mg of solid.

### Example 4: Synthesis of material S3

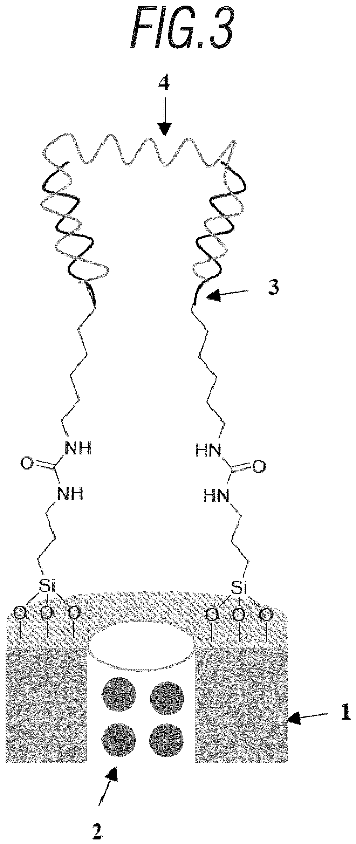

100 µg of material S2 were suspended in 297.5 µl of an aqueous buffer solution at pH 7.5 (MgCl₂ 37.5 mM, Tris-HCI 20 mM), containing oligonucleotide O2 (SEQ No. 2) (5'-TTT TGG GGG GTT GAG AAG GAT CTT TCC ATT GAT GGG GTT TT-3') in a concentration of 2.50 x 10⁻⁶ nmol/mg of solid, and left under stirring at room temperature for 2 hours. Next, the suspension was centrifuged for 2 min at 12,000 rpm and the liquid was separated, washing the solid with 500 µg of the aqueous buffer solution at pH 7.5 (MgCl₂ 37.5 mM, Tris-HCl 20 mM). This suspension was separated again by centrifugation, thus obtaining material S3. Figure 3 shows the diagram of material S3, which was characterised by using thermogravimetry, TEM and X-ray Powder Diffraction techniques. As mentioned earlier, this material S3 was prepared using material S2 loaded with a dye/fluorophore (Rhodamine B) (2) and functionalised with oligonucleotide O1 (SEQ No. 1((3), whereon oligonucleotide O2 hybridises (SEQ No. 2: 5'-TTT TGG GGG GTT GAG AAG GAT CTT TCC ATT GAT GGG GTT TT-3') (4). As a consequence of the hybridisation, given the three-dimensional structure and conformation of the hybridised oligonucleotides O1-O2, the pores of the support made of porous material become blocked. The concentration of oligonucleotide O2 in solid S3 is 2.5 x 10⁻⁶ nmoles/mg of solid.

### Example 5: Synthesis of material S4

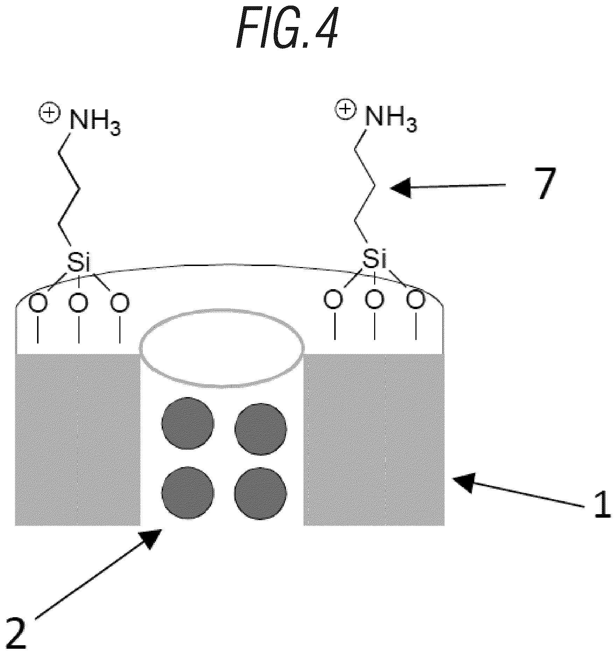

200 µg of calcined MSNs (50-150 nm in diameter, 1200 m²/g of total area), obtained in example 1, were suspended in a solution containing 766.4 mg (0.16 mmol) of Rhodamine B in 10 ml of acetonitrile, leaving the mixture under stirring at room temperature for 24 hours. Next, 293 µl (1.25 mmol) of (3-aminopropyl) triethoxysilane were added and left to react at room temperature for 5.5 hours. Next, the mixture was vacuum filtered, washed with acetonitrile and dried in a heater at 65°C. The solid obtained was characterised by using thermogravimetry and elemental analysis techniques and identified as S4. Figure 4 shows material S4, wherein it can be observed that said porous material is formed by a porous inorganic silicon matrix (1), of the family MCM-41, loaded with a dye/fluorophore (Rhodamine B) (2) and with the outer surface functionalised with a cationic organic group (7), such as 3-aminopropyl) triethoxysilane.

### Example 6: Synthesis of material S5

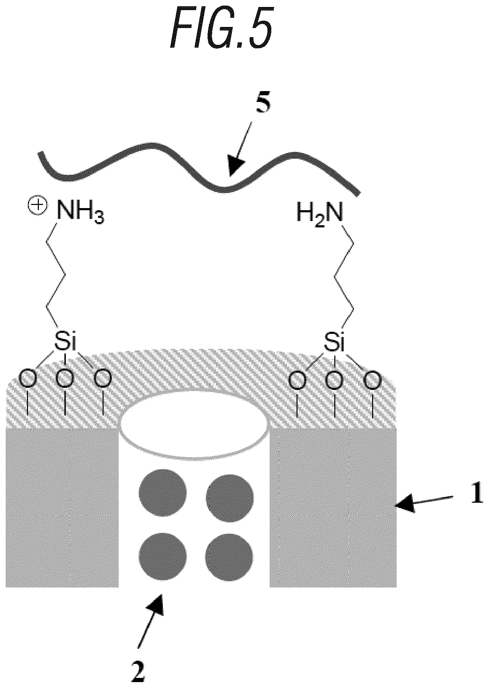

500 µg of material S4 were suspended in 500 µl of an aqueous buffer solution at pH 7.5 (MgCl₂ 37.5 mM, Tris-HCI 20 mM) containing oligonucleotide O3 (SEQ No. 3: 5'-TTG AGA AGG ATC TTT CCA TTG ATG-3') in a concentration of 2.00 x 10⁻⁶ nmol/mg of solid) and left under stirring at 37°C for 30 min. Next, the suspension was centrifuged for 2 min at 12,000 rpm and the liquid was separated, washing the solid with 1 ml of the aqueous buffer solution. This suspension was separated again by centrifugation, thus obtaining material S5, which was characterised by using thermogravimetry, TEM and X-ray Powder Diffraction techniques. Figure 5 shows material S4 (1), which is loaded with a dye/fluorophore such as Rhodamine B (2), and wherein the pores are blocked by adding oligonucleotide O3 (SEQ No. 3: 5'-TTG AGA AGG ATC TTT CCA TTG ATG-3') (5), giving rise to said material S5. The concentration of oligonucleotide O3 in solid S5 is 2.00 x 10⁻⁶ nmoles/mg of solid.

### Example 7: Synthesis of the support made of nanoporous anodised alumina

The mesoporous alumina plates were synthesised by electrochemical anodisation using high purity aluminium plates (99.99%). Prior to anodisation, the aluminium plates were electropolished in a mixture of ethanol and perchloric acid (4:1, v/v) at 20 V for 4 min to remove the roughness of the surface of the metal. Next, they were washed with abundant water and ethanol, and finally air-dried to remove any trace of the acid. The electropolished plates were anodised with the corresponding electrolyte using a two-step anodisation process.^{9,10} The first anodisation was carried out for 20 hours at 40 V with oxalic acid and at 10 V with sulfuric acid. Electrolyte temperature was 2°C in both cases. The resulting material is a nanostructured porous alumina plate with disordered pores, some of which are interconnected. This initial alumina plate was dissolved by means of a mixture of phosphoric acid (0.4 M) and chromic acid (0.2 M) at 70°C for 3 hours, obtaining an aluminium plate with a marked surface. The second anodisation was carried out under the same voltage and temperature conditions as the first, obtaining a nanostructured porous alumina layer with non-interconnected pores. Pore distribution is ordered or disordered depending on whether the electrolyte used is oxalic acid or sulfuric acid, respectively, as shown in Figure 7 a and b, respectively.

### Example 8: Synthesis of material S6

A porous alumina plate approximately 2 cm in diameter and with a pore depth of 8 µm was immersed in a solution containing 766.4 mg (0.16 mmol) of Rhodamine B in 10 ml of acetonitrile, leaving the mixture under stirring at room temperature for 24 hours. Next, 247 µl (1 mmol) of (3-propyl isocyanate) triethoxysilane were added and left to react at room temperature for 5.5 hours. Next, the plate was removed and allowed to dry. Once dry, the plate was divided into 8 pieces approximately 2 mm in diameter. In this way, material S6 was obtained, which was characterised using the thermogravimetry technique. Material S6 is similar to material S1 but, as mentioned earlier, porous alumina was used to obtain it instead of mesoporous silicon.

### Example 9: Synthesis of material S7

Material S6 was immersed in 700 µl of an acetonitrile solution with Rhodamine B (1 mM), adding oligonucleotide O1 (NH₂-(CH₂)₆-5'-AAA AAA CCC CCC-3') in a concentration of 1 nmol/plate. 2 µl of triethylamine were added to this mixture and left under stirring at room temperature for 3 hours. Next, the material was removed and allowed to dry at room temperature, thus obtaining material S7, which was characterised using the thermogravimetry technique. Material S7 is similar to material S2 but, as mentioned earlier, porous alumina was used to obtain it instead of mesoporous silicon.

### Example 10: Synthesis of material S8

Material S7 was immersed in 297.5 µl of an aqueous buffer solution at pH 7.5 (MgCl₂ 37.5 mM, Tris-HCI 20 mM), containing oligonucleotide O2 (5'-TTT TGG GGG GTT GAG AAG GAT CTT TCC ATT GAT GGG GTT TT-3') in a concentration of 2.50 x 10⁻⁶ nmol/mg of solid, and left under stirring at room temperature for 2 h. Next, it was washed with a few drops of the aqueous buffer solution at pH 7.5 (MgCl₂ 37.5 mM, Tris-HCI 20 mM) and allowed to dry at room temperature, thus obtaining material S8, which was characterised using the thermogravimetry the FESEM techniques. Material S8 is similar to material S3 but, as mentioned earlier, porous alumina was used to obtain it instead of mesoporous silicon.

### Example 11: Synthesis of material S9

A porous alumina plate approximately 2 cm in diameter and with a pore depth of 8 µm was immersed in a solution containing 766.4 mg (0.16 mmol) of Rhodamine B in 10 ml of acetonitrile, leaving the mixture under stirring at room temperature for 24 hours. Next, 293 µl (1.25 mmol) of (3-aminopropyl) triethoxysilane were added and left to react at room temperature for 5.5 hours. Next, the plate was removed and allowed to dry. Once dry, the plate was divided into 8 pieces approximately 2 mm in diameter. In this way, material S9 was obtained, which was characterised using the thermogravimetry technique.

### Example 12: Synthesis of material S10

Material S9 was immersed in 297.5 µl of an aqueous buffer solution at pH 7.5 (MgCl₂ 37.5 mM, Tris-HCI 20 mM) containing oligonucleotide O3 (5'-TTG AGA AGG ATC TTT CCA TTG ATG-3') in a concentration of 2.00 x 10⁻⁶ nmoles/plate) and left under stirring at 37°C for 30 min. Next, it was washed with a few drops of the aqueous buffer solution at pH 7.5 (MgCl₂ 37.5 mM, Tris-HCl 20 mM) and allowed to dry at room temperature, thus obtaining solid S10, which was characterised using the thermogravimetry the FESEM techniques.

### Example 13: Characterisation of the materials obtained

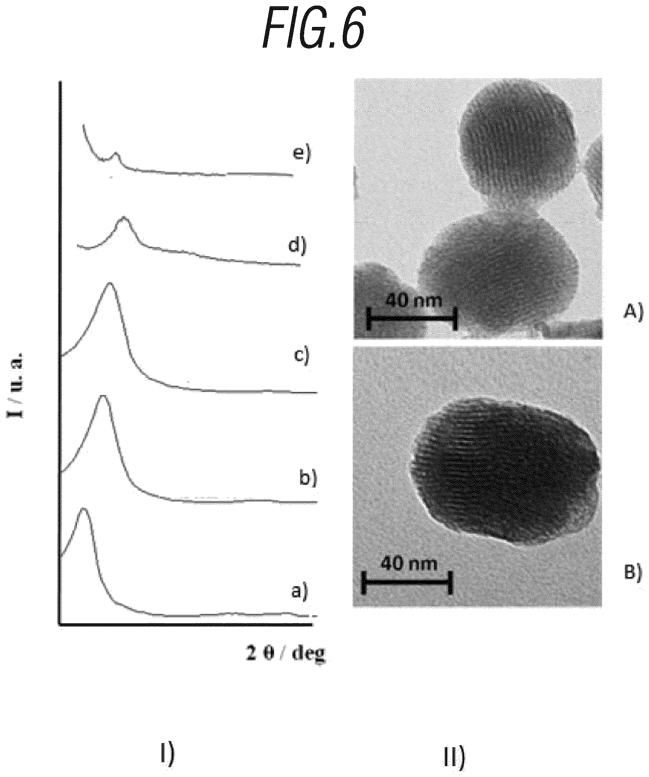

The synthesised materials were characterised using standard methods. Figure 6 shows the characterisation carried out on the different materials obtained. It specifically shows the X-ray powder diffractogram for: a) non-calcined MSNs, b) calcined MSNs, c) material S1 (which is equal to S4), d) material S2, and e) material S3 (equal to S5), and II) shows Transmission Electron Microscopy images for: A) calcined MSNs and B) material S3, S5 showing the spherical morphology typical of nanoparticles. The non-calcined MSNs support shows the 4 typical reflections of MCM-41-type materials corresponding to the directions in the plane (100), (110), (200) and (210). A displacement of the peak (100) can be observed in the diffractogram of calcined MSNs, corresponding to a cell contraction of approximately 7 A. Lastly, in the following diffractograms (S1, S2, S3, materials S4 and S5), the peak intensity value (100) proves that the process of filling the pores with the indicator species, in these cases the dye/fluorophore, and the functionalisation of the surface of the mesoporous solid do not destroy the three-dimensional structure of the MSNs material. The spherical morphology of the materials prepared can be visualised through Transmission Electron Microscopy (see also Figure 6). The corresponding organic matter content (3-propyl isocyanate, 3-aminopropyl, Rhodamine B and oligonucleotides) present in the different materials was determined by means of thermogravimetric analysis. The values obtained in mmol/mg of SiO₂ are shown in Tables 1 and 2.

**Table 1**

| 3-propyl isocyanate | | Rhodamine B | O1 | O2 |
|---|---|---|---|---|
| **S1** | 3.05 x 10⁻² | 9.84 x 10⁻¹ | - | - |
| **S2** | 3.05 x 10⁻² | 3.16 x 10⁻¹ | 3.18 x 10⁻² | - |
| **S3** | 3.05 x 10⁻² | 3.17 x 10⁻² | 3.18 x 10⁻² | 5.04 x 10⁻⁵ |

**Table 2**

| 3-aminopropyl | | Rhodamine B | O3 |
|---|---|---|---|
| **S4** | 1.23 x 10⁻¹ | 4.44 x 10⁻⁴ | - |
| **S5** | 1.23 x 10⁻¹ | 3.79 x 10⁻⁴ | 3.32 x 10⁻⁴ |

The synthesised solids S6-S10 were characterised using the usual techniques for this type of systems. Figure 7 shows the FESEM (Field Emission Scanning Electron Microscopy) image for the initial support made of nanoporous anodised alumina and for solid S8. The corresponding organic matter content (3-propyl isocyanate, 3-aminopropyl, Rhodamine B and oligonucleotides) present in the different materials was determined by means of thermogravimetric analysis. The values obtained in mmol/mg AlO₂ are shown in Tables 3 and 4.

**Table 3**

| 3-propyl isocyanate | | Rhodamine B | O1 | O2 |
|---|---|---|---|---|
| **S6** | 1.1 x 10⁻² | 6.7 x 10⁻¹ | | |
| **S7** | 1.1 x 10⁻² | 25 x 10⁻¹ | 1.0 x 10⁻¹ | |
| **S8** | 1.1 x 10⁻² | 2.4 x 10⁻¹ | 1.0 x 10⁻¹ | 1.1 x 10⁻¹ |

**Table 4**

| 3-aminopropyl | | Rhodamine B | O3 |
|---|---|---|---|
| **S9** | 1.2 x 10⁻¹ | 5.47 x 10⁻² | - |
| **S10** | 1.2 x 10⁻¹ | 3.79 x 10⁻² | 4.52 x 10⁻² |

### Example 14: Behaviour of materials S3 and S5 in the presence of the genomic DNA of Candida albicans.

Figure 8a) shows the functional diagram of material S3 (1). 100 µg of material S3 were suspended in 400 µl of the buffer solution at pH 7.5 (MgCl₂ 37.5 mM, Tris-HCI 20 mM). 200 µl were pipetted from this suspension and 800 µl of the buffered aqueous solution were added. No release of the indicator species (dye/fluorophore) (2) was observed over time. However, upon repeating the experience in the presence of genomic DNA of *Candida albicans* (6) (in a concentration of 2.5 x 10⁻⁵ nmol/mg of solid), a release of the indicator species (dye/fluorophore) (2) was observed, as shown in Figure 9a.

In the presence of genomic DNA of *Candida albicans* (2 x 10⁻⁵ and 4 x 10⁻⁶ nmoles/mg of solid for S3 (8a) and S5 (8b), respectively) in a buffer solution at pH 7,5 (MgCl₂ 37,5 mM, Tris-HCI 20 mM), the opening of the "molecular gates" takes place, with the ensuing release of the dye/fluorophore (2). Materials S8 and S10 behave similarly.

Similarly, figure 8b) shows the functional diagram of material S5. In this experiment, 500 µg of material S5 (1) were suspended in 400 µl of the buffer solution at pH 7.5 (MgCl₂ 37.5 mM, Tris-HCI 20 mM). Of this suspension, 200 µl were pipetted and 800 µl of the buffer solution were added without observing a release of the indicator species (dye/fluorophore) (2) over time at 37°C (see Figure 8B). However, upon repeating the experience in the presence of genomic DNA of *Candida albicans* (in a concentration of 4 x 10⁻⁶ nmol/mg of solid) (6), a release of the indicator species (dye/fluorophore) (2) was observed, as shown in Figure 9b.

As observed earlier, the massive release of dye/fluorophore (Rhodamine B) is caused by the opening of the "molecular gate" upon hybridisation of the oligonucleotides (O2 and O3) situated on the different materials with the genomic DNA of *Candida albicans* present in the sample studied.

### Example 15: Behaviour of materials S8 and S10 in the presence of genomic DNA of Candida albicans.

Material S8 was immersed in 1 ml of the buffer solution without observing a release of the indicator species over time at room temperature. However, upon repeating the experience in the presence of genomic DNA of *Candida albicans* (in a concentration of 2.5 x 10⁵ nmol/mg of solid), a release of the indicator species was observed, as shown in Figure 10a.

Similarly, material S10 was immersed in 1 ml of the buffer solution without observing any release of the indicator species over time at 37°C, as opposed to what was observed in the presence of genomic DNA of *Candida albicans* (in a concentration of 4 x 10⁻⁶ nmol/solid). In this case, a significant release of the indicator species (dye/fluorophore) was observed, as shown in Figure 10b.

As observed, both materials are unable to release dye/fluorophore (Rhodamine B) in the absence of genomic DNA of *Candida albicans.* On the contrary, a massive release of Rhodamine B is observed in the presence of genomic DNA due to the opening of the "molecular gate", upon hybridisation of the oligonucleotides (O2 and O3) situated on the different materials with the genomic DNA provided by *Candida albicans.*

### Example 16: Behaviour of material S8 in the presence of samples infected by Candida albicans from infected patients.

As observed in Figure 11, in the presence of *Candida albicans* in real samples of infected patients in different competitive media (cerebrospinal fluid (A) and peritoneal fluid (B)), material S8 is capable of releasing the dye/fluorophore (Rhodamine B), producing significant fluorescent intensity. On the contrary, release of Rhodamine B is not observed in the absence of *Candida albicans.*

### Example 17: Behaviour of material S8 in the presence of DNA of other strains of Candida and of other infectious pathogens

Figure 12 shows Rhodamine B released from material S8 in the presence of different pathogens and other strains of *Candida.* As observed, Rhodamine B is only released in the presence of *Candida albicans.* This experience demonstrates the high selectivity achieved with material S8.

## Claims

1. A porous material for detecting *Candida albicans,* which comprises an indicator species in the inner pores and at least one DNA sequence anchored on the outer surface thereof which is complementary to a fragment of the *Candida albicans* genome, wherein the complementary DNA sequence blocks the release of the indicator species from the inner pores of the support.

2. The porous material, according to claim 1, wherein the complementary DNA sequence is bonded to the outer surface of the support by means of a neutral organic group and a bonding oligonucleotide.

3. The porous material, according to claim 2, wherein the neutral organic group is selected from carboxylic acid (-COOH), alcohol (-OH), aldehyde (-CHO), alkene, alkyne, amine (-NH₂ or -NR'R"), amide (-C(O)NR'R"), azide (-N₃), ketone (-C=O), ester (-COOR'), ether (R'-O-R"), halogen, imine (RR'C=NR"), isocyanate (-NCO), isothiocyanate (-N=C=S), nitrile (-C=N), nitro (-NO₂) or thiol (-SH).

4. The porous material, according to claim 3, wherein the bonding oligonucleotide is oligonucleotide O1 (SEQ No. 1).

5. The porous material, according to claim 4, wherein oligonucleotide O1 (SEQ No. 1) is hybridised with oligonucleotide O2 (SEQ No. 2).

6. The porous material, according to claim 1, wherein the complementary DNA sequence is bonded to the outer surface of the support by means of a cationic group.

7. The porous material, according to claim 6, wherein the cationic organic group is selected from amines, guanidine groups (H-N=(NHR)NH2), phosphonium (PH₄⁺) or quaternary ammonium (NR₄⁺).

8. The porous material, according to claim 7, wherein the complementary DNA sequence is oligonucleotide O3 (SEQ No. 3).

9. The porous material, according to any one of claims 1 to 8, **characterised in that** it consists of mesoporous silicon dioxide with a specific surface of between 200-1400 m²g⁻¹.

10. The porous material, according to any one of claims 1 to 8, **characterised in that** it consists of a film or plate made of nanoporous anodised alumina.

11. An in vitro diagnostic method for detecting *Candida albicans* which comprises:
a) putting the porous material of claim 1 in contact with the DNA sample to be analysed, in an aqueous solution,
b) incubating the mixture of step a) so as to produce the hybridisation reaction,
c) measuring the amount of indicator species released into the aqueous phase of the mixture.

12. A method for preparing the porous material of claim 1, **characterised in that** it comprises the following steps:
a) Preparing a support made of porous silicon dioxide or nanoporous anodised alumina,
b) Introducing the indicator species into the inner pores of the substrate prepared in step a),
c) Functionalising the loaded substrate obtained in step b) by bonding a neutral or cationic organic group to the surface of said substrate,
d) If necessary, derivatising the substrate with a bonding oligonucleotide bonded to the neutral organic group,
e) Adding a DNA sequence which is complementary to a fragment of the *Candida albicans* genome to the porous inorganic support obtained in step c) or d).

13. The method for preparing the porous material according to claim 12, **characterised in that** a DNA sequence that is hybridised with the DNA sequence which is complementary to a fragment of the genome of *Candida albicans* is added to the support obtained in step e).
